Europäisches Patentamt

European Patent Office

Office européen des brevets

(11) Publication number: **0 347 879**

**A1**

## (12) EUROPEAN PATENT APPLICATION .

(21) Application number: **89111293.0**

(22) Date of filing: **21.06.89**

(51) Int. Cl.⁴: **G01N 33/566 , G01N 33/535 , G01N 33/52** .

(30) Priority: **22.06.88 IT 4812088**

(43) Date of publication of application:
**27.12.89 Bulletin 89/52**

(84) Designated Contracting States:
**AT BE CH DE ES FR GB GR IT LI LU NL SE**

(71) Applicant: **FIDIA S.p.A.**
**Via Ponte della Fabbrica 3-A**
**I-35031 Abano Terme (Padova)(IT)**

(72) Inventor: **Della Valle, Francesco**
**Via Cerato 14** .
**Padova(IT)**
Inventor: **Kirschner, Gunter**
**Via Leonardo da Vinci 14**
**35031 Abano Terme Padova(IT)**

(74) Representative: **Vossius & Partner**
**Siebertstrasse 4 P.O. Box 86 07 67**
**D-8000 München 86(DE)**

(54) **Method for the quantitative determination of gangliosides of the ganliotetraose series by cholera toxin conjugated with an enzyme.**

(57) A method is disclosed for the quantitative determination of gangliosides of the gangliotetraose series characterized by the use of a reagent consisting of the subunit B of cholera toxin conjugated with a peroxidase, preferably horseradish peroxidase.

EP 0 347 879 A1

# METHOD FOR THE QUANTITATIVE DETERMINATION OF GANGLIOSIDES OF THE GANGLIOTETRAOSE SERIES BY CHOLERA TOXIN CONJUGATED WITH AN ENZYME

The present invention relates to a method for the quantitative determination of gangliosides, particularly those of the gangliotetraose series. The method is characterized by the use of a reagent constituted of cholera toxin conjugated with an enzyme, particularly the subunit B conjugated with a peroxidase.

The ever-growing importance of gangliosides in many biological processes increases the need for simple, fast and reliable analytical methods for their determination. Since it is frequently necessary to determine the ganglioside content in small samples which are often difficult to obtain, fundamental requisites are sensitivity of the method and the possibility to carry out a large number of analyses simultaneously and quickly.

Recently various ways have been suggested of overcoming these problems, using the technique of Magnani et al., in combination with TLC separation.

Determination of gangliosides can be effected, for example, either using specific antisera bound with horseradish peroxidase or labelled with $^{125}$I (Kolsto-Otnaess A.-B., Laegreid A.: Detection of femtomolar quantities of the ganglioside $GM_1$ on thin-layer chromatography plates by native choleratoxin and labelled antisera. Current Microbiol. 13, 323-326, 1986), labelled antibodies (Higashi H., Fukui Y., Ueda S., Kato S., Hirabayashi Y., Matsumoto M. and Naiki M.: Sensitive enzyme-immunostaining and desitometric determination on thin-layer chromatography of N-glycolylneuraminic acid-containing glycosphingolipids, hanganutziudeicher antigens. J. Biochem. 95, 1517-1520, 1984; Harpin, 1985), the use of the cholera toxin and specific anticholera toxin antisera (Kolsto-Otnaess A.-B., Laegreid A.: Detection of femtomolar quantities of the ganglioside $GM_1$ on thin-layer chromatography plates by native choleratoxin and labeled antisera. Current Microbiol. 13, 323-326, 1986), radioactively labelled conjugated with HRP or with alkaline phosphatase.

US-A-4,469,795 discloses a radioassay test for the determination of the concentration of monosialoglycosphingolipid $GM_1$ ganglioside. In the test, a solution of $^{125}$Iodinated cholera toxin or B subunit is prepared, and a radioassay is performed on a solution containing $GM_1$ ganglioside.

Another method consists in transforming the gangliosides by enzymatic reaction into asialogangliosides and subsequent determination by antiasialoganglioside antibodies (Hirabayashi).

The present invention now provides a method which, by using one easily handled reagent, makes it possible to quantitatively determine ganglioside $GM_1$ directly in biological samples. The method is particularly characterized by the use of a reagent constituted by subunit B of cholera toxin conjugated with peroxidase, particularly horseradish peroxidase. In addition to the determination of $GM_1$, the method is further useful for the quantitative determination of other gangliosides of the gangliotetraose series by first subjecting such other gangliosides to enzymatic hydrolysis to convert those gangliosides to $GM_1$, and then detecting the formed $GM_1$.

The method of the present invention provides a means for quantitative detection of $GM_1$ gangliosides by the use of one easily handled reagent. $GM_1$ is one species of known gangliosides and is one member of the gangliotetraose series. According to a further aspect of the invention, quantitative determination of other gangliosides of the gangliotetraose series can be accomplished by first converting such other gangliosides to $GM_1$, and then detecting the amount of $GM_1$ by the method of the invention.

The process of the present invention is importantly characterized, in that:

    (a) the process uses only one reagent;

    (b) the process uses a non-reactive agent, thereby avoiding the use of antibodies or reactive derivatives; and

    (c) the process can be used with automated systems.

These important characteristics permit the process of the invention to be operated easier and less expensively than prior art procedures. As compared to prior procedures, the process of the invention is simplified by the use of only one non-reactive reagent, and is also more reliable.

It is also an important aspect of the invention that $GM_1$ can be quantitatively detected in biological samples. The following, therefore, is a description of a preferred embodiment of the invention for a determination of gangliosides in a biological sample.

Preparation of a standard concentration curve for the assay method of the invention utilizes $GM_1$ gangliosides, which is commercially available (for example, through Fidia S.p.A., Abano Terme, Italy). The important single reagent of the invention methods comprises the subunit B of cholera toxin conjugated with a peroxidase, preferably with horseradish peroxidase.

It is known that gangliosides, particularly $GM_1$, interact with choleratoxin and inactivate the toxin's biological activities. Recent studies have particularly shown that the enterotoxin from Vibrio cholerae, which is responsible for the gastrointestinal manifestation of clinical cholera, binds very strongly to gangliosides, thereby blocking the biological effects of cholera toxin. This known affinity of gangliosides for cholera toxin has been utilized in a method for the purification of cholera toxin by affinity chromatography utilizing an insoluble polysaccharide, e.g. agarose, derivatives comprised of covalently linked gangliosides; see US-A-4,225,487.

Cholera toxin, the holotoxin produced by certain strains of Vibrio cholerae, has been shown to be composed of two protein subunits, known as A and B subunits. Subunit A is responsible for epithelial cell penetration and the enzymatic effect leading to net loss of fluid in the gut lumen. Subunit B, on the other hand, binds the toxin to monosialosylganglioside $GM_1$ receptor sites on the cell wall, appears to possess no toxic activity and is highly immunogenic.

Subunits A and B have been commercially available, and, in fact, the DNA sequences coding for the subunits have been sequenced and the subunits can now be prepared by recombinant DNA technology; see US-A-4,666,837.

## Preparation of biological samples

Gangliosides are first extracted from a biological sample according to known methods. Various methods are known in the art and can be utilized in conjunction with the present invention. However, the preferred extraction method is that of Tettamanti et al. (B.B.A. 296, 160 (1973)). According to this method, gangliosides are extracted from a biological sample, such as serum, plasma or cephalorhachidian fluid. Necessary quantities for analysis can vary, but the minimum quantities necessary according to the Tettamanti et al. procedure are 1-2 ul for serum or plasma, and 10-20 ul for cephalorhachidian fluid.

## Quantitative determination of $GM_1$

A standard concentration curve is first prepared by the following procedure.

Polystyrene plates for microtitration are coated with 50 ul of a solution of 1-5 mg of $GM_1$ dissolved in 100 ml of $EtOH/H_2$ 1:1 for 90 min. at room temperature. The plates are washed three times with water and then left full of water for at least 12 hours at 4°C.

Aspecific bond sites are blocked with a solution (4 mg/ml) of bovine serum albumin in phosphate buffer (1 mg/ml) [BSA/PBS] for 90 minutes at room temperature. Reference plates without $GM_1$ are prepared in the same way.

The subunit B of choleratoxin is conjugated with a peroxidase, preferably with horseradish peroxidase (HRP), to prepare the conjugated reagent (B-HRP) for the assay of the invention. The conjugate, B-HRP, is per se known and commercially available.

A standard curve is then prepared by dissolving a suitable quantity of $GM_1$ in BSA/PBS. At regular intervals 5 $\mu l$ of a solution in BSA/PBS of the conjugated (B-HRP) is added in standard samples in a ratio of 1:5.

The solution is left to incubate for 45 minutes at room temperature after which 50 $\mu l$ of the same are added to the microtitration plates coated with $GM_1$ and then left to react for another 45 minutes at room temperature. To accomplish a colorimetric reaction for ultimate detection by photometry, the plates are washed with PBS and distilled water, alternately, 50 $\mu l$ are added of an O-phenylenediamene (OPD) reagent (25 mg dissolved in 5 ml of absolute ethanol and 1 ml of the resulting solution is diluted with 50 ml of PBS and 5 ul of 30% oxygenated water) and it is left to react for 15 minutes at room temperature. The colorimetric reaction is blocked by adding 50 $\mu l$ of $H_2SO_4$ 2.5 M and extinction is read with a photometer for optical density readings for microElisa plates at 492 nm against a blank constituted by the substrate and by the blocking solutions. The blank values are subtracted, to thereby prepare a standard concentration curve.

The same procedure described above for the standard curve is followed for the biological samples which are obtained as described previously, diluting the samples, where necessary, in BSA/PBS in order to obtain a concentration coming within the linear range of the standard curve. Comparison to the standard concentration curve then permits a determination of the $GM_1$ concentration in the biological samples.

## Quantitative determination of gangliosides after thin layer chromatographic separation

As described above, the assay method of the invention can also be used for the determination of other gangliosides of the gangliotetraose series by first converting those other gangliosides to $GM_1$ ganglioside. Such gangliosides can be converted to $GM_1$ by subjecting the gangliosides, or a sample containing the gangliosides, to enzymatic hydrolysis. Particularly useful is enzymatic hydrolysis with neuroaminidase, such as Vibro cholerae neuraminidase (Calbiochem) (See, for example, Li et al., Ad. Exp. Ther. Biol. 125, 93 (1981)).

According to this method, a sample containing gangliosides is first subjected to thin layer chromatography to isolate gangliosides, the plate is then subjected to enzymatic hydrolysis and determination of GM₁ is conducted as described above using the conjugated B-HRP reagent.

More particularly, thin layer chromatography of gangliosides is carried out on high performance thin layer chromatography plates under the following conditions: temperature 18-20°C, solvent chloroform/methanol/0.2%, CaCl₂ 50:42:11 (v/v/v), chromatographic run of 1 hour. On each plate are placed standard amounts of GM₁ corresponding to 0.05, 0.1, 0.2, 0.4, and 0.8 ng of GM₁ and the samples to be analyzed.

After chromatography the plate is dried and then immersed twice, being dried between immersions, in a solution obtained by diluting 8 ml of a solution of 3 g of polyisobutylmethacrylate in 100 ml of chloroform with 50 ml of n-hexane. The plate thus fixed is placed in an incubation tank, washed with acetate buffer, 50 mM, pH 5.4, containing 0.04% of CaCl₂.

Enzymatic hydrolysis of the gangliosides is then accomplished by incubating the plates for 90 minutes at 37°C with neuroaminidase (200 mU/ml phosphate buffer). Finally, the plate is washed five times with BSA/PBS and then incubated for 2 hours at room temperature with a solution of B-HRP prepared as described above in "Quantitative Determination of GM₁".

The plate is then washed three times with BSA/PBS, twice with water, incubated for 30 minutes at room temperature and in the dark with a freshly prepared solution of 0-phenylenediamene. Quantification is carried out by a spectrophotodensitometer equipped with an integrator reading absorbency at 440 nm, similar to the procedure described above.

As previously described, enzymatic hydrolysis by neuroaminidase of gangliosides of the gangliotetraose series leads to the formation of one single compound, GM₁ (Li et al., Adv. Exp. Ther. Biol. 125, 93 (1981)) and it is therefore suitable as an analytical method for the determination of these gangliosides. Furthermore, the use of a single reagent (B-HRP) avoids the problems presented by poor stability of the silica gel layer during manipulation with aqueous solutions.

For each batch of B-subunit of cholera toxin, the optical dilution in BSA/PBS should be determined, in order to have a good signal/noise ratio in the photodensitometric reading and to compensate for the fact that the derivatization of B-subunit is not altogether constant.

In order to verify the constancy of the photodensitometric response for the various gangliosides, determinations are made of known quantities of GM₁, GD₁ₐ, GD₁ᵦ, GT₁ᵦ and GQ₁ᵦ, individual doses being within a range of 0.01 and 2.0 ng.

The plates are eluted and developed as previously described and photodensitometric readings are made. The results indicate that the various gangliosides examined after enzymatic treatment give the same response as GM₁.

To further verify a possible cross reaction between GM₁ and other gangliosides (Cuman et al., Mol. Cell Biochem. 46, 155 (1982)), pure gangliosides were analyzed without neuroaminidase treatment and it was confirmed that only GD₁ᵦ reacts with unit B of the cholera toxin with a relative response of 0.1 with regard to GM₁.

Should one wish to determine GM₁ alone, once it has been ascertained that the possible GD₁ᵦ ganglioside does not interfere significantly with determination, it is advisable to use microtitration as an analytical system, since this allows for a rapid dosage of a large number of samples. Care must be taken that not only the sera, but also the liquors are deproteinized because falsely higher values are obtained in the case of non-deproteinized liquor.

All of the above procedures can also be easily performed with automatic analyzer systems available for use in automatic enzyme immunological assays, such as Labsystems EIA2 automatic analyzer.

The process of the present invention, as noted above, has excellent reliability and performs with a detection limit of 50 fmol and a coefficient of variation of 3.0%.

## Claims

1. A method for quantitative analytical determination of gangliosides of the gangliotextraose series which comprises:

a) subjecting a sample containing gangliosides to enzymatic hydrolysis to convert gangliosides of the gangliotetraose series in said sample to GM₁ ganglioside;

b) incubating the hydrolyzed sample with a conjugate consisting of the subunit B of cholera toxin conjugated with a peroxidase; and

c) detecting GM₁ in the sample by photometric measurement.

2. A method for quantitative analytical determination of GM₁ ganglioside which comprises:

a) incubating a sample containing GM₁ with a conjugate consisting of the subunit B of choleratoxin conjugated with a peroxidase; and

b) detecting GM₁ in the sample by photometric measurement.

3. The method according to any one of claims 1 or 2, wherein said photometric measurement comprises:

a) subjecting said incubated sample to a colorimetric reaction with a colorimetric reagent; and

b) measuring the extinction of said sample by spectrophotodensitometry.

4. The method according to any one of claims 1 to 3, wherein said spectrophotodensitometry is conducted at 440 or 492 nm.

5. The method according to any one of claims ·1 to 4, wherein said peroxidase ie horseradish peroxidase.

6. A method for quantitative analytical determination of gangliosides of the gangliotetraose series which comprises:

a) subjecting a sample containing gangliosides to thin layer chromatography;

b) subjecting said chromatographed sample to enzymatic hydrolysis to convert gangliosides of the gangliotetraose series in said sample to $G_{M1}$ ganglioside;

c) incubating the hydrolyzed sample with a conjugate consisting of the subunit B of cholera toxin conjugated with a peroxidase;

d) subjecting said incubated sample to a colorimetric reaction with a colorimetric reagent; and

e) measuring the extinction of said sample by spectrophotodensitometry.

7. The method according to any one of claims 1 to 6, wherein the enzyme for said enzymatic hydrolysis is neuraminidase and said peroxidase is horseradish peroxidase.

8. The method according to any one of claims 6 or 7, wherein said spectrophotodensitometry is conducted at 440 nm.

9. The method according to any one of claims 6 to 8, wherein said chromatography is conducted in a solvent system of chloroform/methanol/0.2% $CaCl_2$, 50:42:11 (v/v/v), at a temperature of 18-20°C and for about one hour.

10. The method according to any one of claims 6 to 9, wherein after said chromatography, said sample is fixed by immersion in a solution comprising polyisobutylmethacrylate, chloroform and hexane.

11. The method according to any one of claims 1 to 10, wherein said colorimetric reagent is O-phenylenediamine.

**EUROPEAN SEARCH REPORT**

European Patent Office

| DOCUMENTS CONSIDERED TO BE RELEVANT | | | EP 89111293.0 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,A | US - A - 4 469 795 (GINNS et al.) * Abstract; claims 1,2 * | 1,2 | G 01 N 33/566 G 01 N 33/535 G 01 N 33/52 |
| P,A | CHEMICAL ABSTRACTS, vol. 109, no. 25, December 19, 1988, Columbus, Ohio, USA M. YAMAZAKI et al.: "Ganglioside GM1 or other component determination based on peroxidase reactions." page 434, abstract no. 226264v & Jpn. Kokai Tokkyo Koho JP 63 06,462 (88 06462) | 1-5 | |
| A | CHEMICAL ABSTRACTS, vol. 103, no. 11, September 16, 1985, Columbus, Ohio, USA M. SAITO et al.: "In situ immunological determination of basic carbohydrate structures of gangliosides on thin-layer plates." page 320, abstract no. 84523d & Anal.Biochem. 1985, 148(1), 54-8 | 1,6 | |

| | TECHNICAL FIELDS SEARCHED (Int. Cl.4) |
|---|---|
| | G 01 N 33/00 |

The present search report has been drawn up for all claims

| Place of search | Date of completion of the search | Examiner |
|---|---|---|
| VIENNA | 22-09-1989 | SCHNASS |